(19) Europäisches Patentamt
European Patent Office
Office européen des brevets

(11) **EP 4 086 624 A1**

(12) **EUROPEAN PATENT APPLICATION**

(43) Date of publication:
**09.11.2022 Bulletin 2022/45**

(21) Application number: **22172383.6**

(22) Date of filing: **09.05.2022**

(51) International Patent Classification (IPC):
**G01N 33/28** (2006.01)    **C11B 9/00** (2006.01)
**B01D 17/02** (2006.01)

(52) Cooperative Patent Classification (CPC):
**G01N 33/28; B01D 17/02; A61Q 13/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.05.2021 US 202163185655 P**

(71) Applicant: **Colgate-Palmolive Company**
**New York, NY 10022 (US)**

(72) Inventors:
 • **CHEN, Changlong**
 **New Brunswick, 08901 (US)**

 • **SHEN, Hongwei**
 **Holmdel,, 07733 (US)**
 • **JOGUN, Suzanne**
 **Wayne,, 07470 (US)**
 • **LESNIAK, Ewelina**
 **Linden,, 07036 (US)**
 • **KOZUBAL, Cheryl**
 **Somerset, NJ, 08873 (US)**
 • **MOONEY, Douglas**
 **East Brunswick,, 08816 (US)**

(74) Representative: **Wichmann, Hendrik**
**Wuesthoff & Wuesthoff**
**Patentanwälte PartG mbB**
**Schweigerstraße 2**
**81541 München (DE)**

(54) **METHODS FOR DETERMINING EQUIVALENT ALKANE CARBON NUMBER**

(57)    Disclosed herein are methods to determine the equivalent alkane carbon number (EACN) of a composition comprising an oil.

4.6%    4.8%    5.0%    5.2%    5.4%    5.6%

wt. % NaCl

FIG. 1

**Description**

**CROSS-REFERENCE TO RELATED APPLICATION**

This application claims the benefit of priority from U.S. Provisional Application No. 63/185,655, filed May 7, 2021, the contents of which are hereby incorporated herein by reference in their entirety.

**BACKGROUND**

Fragrances are typically formulated using odoriferous essential oils and surfactant. Discovery of formulations which provide for adequate physico-chemical properties may be onerous. Having a method to predict the physico-chemical properties of fragrance oil compositions would be beneficial.

Equivalent alkane carbon number (EACN) is the number of carbon atoms in a linear alkane that exhibits the same microemulsion behaviors as the oil in the formulation. For example, n-hexane has an EACN of 6, while cyclohexane has an EACN of 2.2. If compared to log P, which measures how hydrophilic or hydrophobic a molecule is, EACN is more sensitive to the relative polarity of oils. Therefore, EACN is a preferable indicator for predicting physico-chemical properties of oil containing compositions.

Historically, in order to determine the EACN for a fragrance composition, anionic surfactant dihexyl sodium sulfosuccinate and nonionic ethoxylated alcohols, such as C6E4, C8E4, C10E4, and C10E5, are normally used as reference surfactants by creating middle phase microemulsions with fragrance oils. However; due to the polarity of fragrance oil, a single surfactant might not be able to formulate middle phase microemulsions with fragrances. Therefore, a "supporting oil", e.g., isopropyl myristate or limonene, is normally mixed with fragrance to facilitate formation of middle phase microemulsions. Upon formation of middle phase microemulsions, EACN may then be calculated based on the ideal-mixing rule. Significantly, because fragrance compositions are a complex mixture containing ingredients of different species (such as aldehydes, aromatics, and alkanes), mixing with "supporting oil" may not necessarily follow the ideal-mixing rule, thus providing for an inaccurate fragrance EACN value.

There exists a need for methods which provide accurate and predictable EACN values for compositions comprising oil, such as, for example, fragrance oils.

**BRIEF SUMMARY**

This summary is intended merely to introduce a simplified summary of some aspects of one or more implementations of the present disclosure. Further areas of applicability of the present disclosure will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

Applicants have discovered that utilization of an alkoxy sulfate having the Formula (I):

$$\text{Formula (I): } C_a\text{-}(PO)_b\text{-}(EO)_c\text{-}SO_4Na$$

where a is from 6 to 18, b is from 1 to 12, and c is from 0 to 4, within fragrance oil compositions provides for predictive determination of equivalent alkane carbon number (EACN). Furthermore, methods for determining the EACN of a composition comprising fragrance oil and $C_8\text{-}(PO)_4\text{-}EO\text{-}SO_4Na$ are herein described.

Thus, in one aspect, the invention provides for a method for determining the equivalent alkane carbon number (EACN) of a composition comprising oil, the method comprising mixing one or more compositions comprising a compound having Formula (I) $C_a\text{-}(PO)_b\text{-}(EO)_c\text{-}SO4Na$, where a is from 6 to 18, b is from 1 to 12, and c is from 0 to 4; one or more oils; water; and salt; determining the salt concentration which produces an equal volume of oil and volume of water within a formed microemulsion; and determining the equivalent alkane carbon number using Formula (II) HLD = ln(S) - K · EACN + Cc - f(A) - $\alpha_T\Delta$(T); where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta$(T) is the temperature deviation from 25 °C. In certain embodiments, the salt is selected from sodium chloride, potassium chloride, ammonium chloride, calcium chloride, magnesium chloride, or a mixture thereof. In certain embodiments, the salt is sodium chloride. In certain embodiments, the salt is present at an amount of about 1 wt. % to about 10 wt. %. In certain embodiments, the salt is present at an amount of about 10 wt. % to about 25 wt. %. In certain embodiments, the one or more oils are non-polar. In certain embodiments, the one or more oils comprise a polar oil and a non-polar oil. In certain embodiments, the one or more oils comprises a solubilizer. In certain embodiments, the one or more oils are fragrant oils. In certain embodiments, the one or more oils comprises a water soluble solvent. In certain embodiments, a is from 7 to 9, b is from 3 to 5, and c is from 1 to 2. In certain embodiments, a is 8, b is 4, and c is 1.

In other aspects, the invention is a method for determining the equivalent alkane carbon number (EACN) of a composition comprising oil, the method comprising mixing one or more compositions consisting essentially of a compound having Formula (I) $Ca-(PO)_b-(EO)_c-SO_4Na$, where a is from 6 to 18, b is from 1 to 12, and c is from 0 to 4, one or more oils; water; and salt; determining the salt concentration which produces an equal volume of oil and volume of water within a formed microemulsion; and determining the equivalent alkane carbon number using Formula (II) $HLD = \ln(S) - K\ EACN + Cc - f(A) - \alpha_T \Delta(T)$; where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C. In certain embodiments, the salt is selected from sodium chloride, potassium chloride, ammonium chloride, calcium chloride, magnesium chloride, or a mixture thereof. In certain embodiments, the salt is sodium chloride. In certain embodiments, the salt is present at an amount of about 1 wt. % to about 10 wt. %. In certain embodiments, the salt is present at an amount of about 10 wt. % to about 25 wt. %. In certain embodiments, the one or more oils are non-polar. In certain embodiments, the one or more oils comprise a polar oil and a non-polar oil. In certain embodiments, the one or more oils comprises a solubilizer. In certain embodiments, the one or more oils comprises a water soluble solvent. In certain embodiments, a is from 7 to 9, b is from 3 to 5, and c is from 1 to 2. In certain embodiments, a is 8, b is 4, and c is 1.

Further areas of applicability of the present disclosure will become apparent from the detailed description provided hereinafter. It should be understood that the detailed description and specific examples, while indicating the preferred embodiment of the disclosure, are intended for purposes of illustration only and are not intended to limit the scope of the disclosure.

## BRIEF DESCRIPTION OF THE DRAWINGS

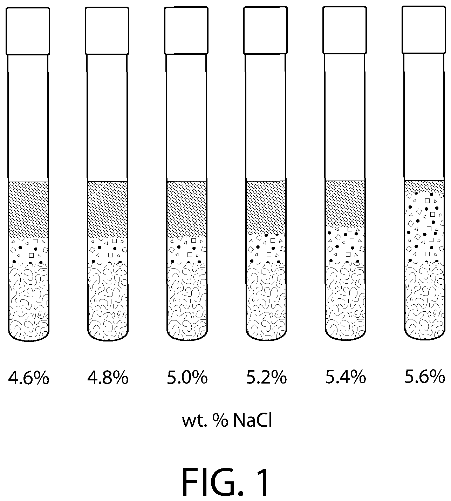

Figure 1 depicts the microemulsion phase behavior of Fragrance "J" with 2 wt. % $SAS-C_8$ solution at various NaCl concentrations.

## DETAILED DESCRIPTION

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other applications and methods. It is to be understood that the invention is not limited in its application to the details of any particular embodiment shown. The terminology used herein is for the purpose of description and not to limit the invention, its application, or uses.

As used herein and in the appended claims, the singular forms "a", "an", and "the" include plural references unless the context dictates otherwise. The singular form of any class of the ingredients refers not only to one chemical species within that class, but also to a mixture of those chemical species. The terms "a" (or "an"), "one or more" and "at least one" may be used interchangeably herein. The terms "comprising", "including", "containing", and "having" may be used interchangeably. The term "include" should be interpreted as "include, but are not limited to". The term "including" should be interpreted as "including, but are not limited to".

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight of the total composition. Reference to a molecule, or to molecules, being present at a "wt. %" refers to the amount of that molecule, or molecules, present in the composition based on the total weight of the composition.

According to the present application, use of the term "about" in conjunction with a numeral value refers to a value that may be +/- 5% of that numeral. As used herein, the term "substantially free" is intended to mean an amount less than about 5.0 weight %, less than 3.0 weight %, 1.0 wt.%; preferably less than about 0.5 wt.%, and more preferably less than about 0.25 wt.% of the composition. As used herein, the term "free", when used with reference to one or more components, means that the cited component(s) is not present in the composition at all. For example, "free of supporting oil" means that no supporting oil is present in the composition.

As used herein, the term "effective amount" refers to an amount that is effective to elicit the desired biological response, including the amount of a composition that, when administered to a subject, is sufficient to achieve an effect toward the desired result. The effective amount may vary depending on the composition, the disease, and its severity and the age, weight, etc., of the subject to be treated. The effective amount can include a range of amounts. As is understood in the art, an effective amount may be in one or more doses, i.e., a single dose or multiple doses may be required to achieve the desired endpoint.

As used herein, the term "physicochemical property" or "physicochemical properties" may refer to physical properties, solvation properties related to interactions with different media, and properties or molecular attributes that define intrinsic chemical reactivity.

Unless defined otherwise, all technical and scientific terms used herein have the same meanings as commonly understood by one of ordinary skill in the art to which this invention belongs. All patents, patent applications, publications, and other references cited or referred to herein are incorporated by reference in their entireties for all purposes. In the event of a conflict in a definition in the present disclosure and that of a cited reference, the present disclosure controls.

The present disclosure is directed towards methods to determine the EACN of a composition comprising one or more oils. In certain embodiments, the methods comprise utilization of a sodium alkoxy sulfate surfactant. The sodium alkoxy sulfate surfactant may have the formula of Formula (I):

$$\text{Formula (I): } C_a\text{-}(PO)_b\text{-}(EO)_c\text{-}SO4Na,$$

where a is from 6 to 18, b is from 1 to 12, and c is from 0 to 4. In certain embodiments, the sodium alkoxy sulfate surfactant has the Formula (Ia) $C_8$-$(PO)_4$-EO-$SO_4$Na (known commercially as Alfoterra K2-41s or Alfoterra 8-41s from Sasol and herein referred to as SAS-$C_8$). The methods provide accurate and predictable EACN values with a wide range of oils, from hydrophilic fragrances to very hydrophobic alkanes. Further, the present inventors have surprisingly and unexpectedly discovered that sodium alkoxy sulfate surfactants, such as SAS-$C_8$, may be utilized to determine EACN values without the need to also use a further supporting oil.

In certain embodiments, the method comprises mixing one or more compositions comprising one or more oils, water, salt, and a compound having Formula (I) $C_a$-$(PO)_b$-$(EO)_c$-SO4Na, where a is from 6 to 18, b is from 1 to 12, and c is from 0 to 4; determining the salt concentration which produces an equal volume of oil and volume of water within a formed microemulsion; and determining the equivalent alkane carbon number using Formula (II) $HLD = \ln(S) - K \cdot EACN + Cc - f(A) - \alpha_T\Delta(T)$; where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

In other embodiments, the method comprises mixing one or more compositions consisting essentially of one or more oils, water, salt, and a compound having Formula (I) $C_a$-$(PO)_b$-$(EO)_c$-$SO_4$Na, where a is from 6 to 18, b is from 1 to 12, and c is from 0 to 4; determining the salt concentration which produces an equal volume of oil and volume of water within a formed microemulsion; and determining the equivalent alkane carbon number using Formula (II) $HLD = \ln(S) - K \cdot EACN + Cc - f(A) - \alpha_T\Delta(T)$; where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

The present invention utilizes a sodium alkoxy sulfate surfactant having the formula of Formula (I): $C_a$-$(PO)_b$-$(EO)_c$-SO4Na, where a is from 6 to 18, b is from 1 to 12, and c is from 0 to 4. In certain embodiments, a is from 6 to 10. In certain embodiments, b is from 1 to 7. In certain embodiments, c is from 1 to 3. In certain embodiments, a is from 6 to 10, b is from 1 to 7, and c is from 1 to 3. In other embodiments, a is from 7 to 9, b is from 3 to 5, and c is from 1 to 2. In further embodiments, a is 8, b is 4, and c is 1.

The invention requires use of salt. Salts useful in the invention include, but are not limited to, NaCl, CaCl$_2$, MgCl$_2$, and KCl, and mixtures thereof. In certain embodiments, the salt is selected from sodium chloride, potassium chloride, ammonium chloride, calcium chloride, magnesium chloride, or a mixture thereof. In certain embodiments, the salt comprises NaCl. In other embodiments, the salt is NaCl. The salt may be present at various amounts or concentrations. In certain embodiments, the salt is present in an amount from about 1 wt. % to about 30 wt. %. For example, salt may be present in an amount of about 1.0 weight%, 2.0 weight %, about 2.5 weight %, about 3.0 weight %, about 3.5 weight %, about 4.0 weight %, about 4.5 weight %, about 5.0 weight %, about 5.5 weight %, about 6.0 weight %, about 6.5 weight %, about 7.0 weight %, about 7.5 weight %, about 8.0 weight %, about 8.5 weight %, about 9.0 weight %, about 9.5 weight %, about 10.0 weight %, about 10.5 weight %, about 11.0 weight %, about 11.5 weight %, about 12.0 weight %, about 12.5 weight %, about 13.0 weight %, about 13.5 weight %, about 14.0 weight % about 15.0 weight %, about 20.0 weight %, about 25.5 weight %, or about 30.0 weight %. In another example, salt may be present in an amount of from about 1.0 % to about 10.0%, from about 2.0% to about 6.0%, from about 4 wt. % to about 6 wt. %, about 10.0% to about 30.0%, from about 10% to about 25%, about 15.0% to about 30.0%, or about 5.0% to about 28.0%, based on the weight of the composition. In further embodiments, salt is present in an amount of about 1.0% or more, about 4.0% or more, about 10.0% or more, or about 15.0% or more up to about 30.0%, based on the weight of the composition. In further embodiments, salt is present in an amount of about 3.5% to 5.5%, about 5.0% to about 15.0%, about 10.0% to about 20.0%, or about 2.0% to about 20.0%, based on the weight of the composition.

A microemulsion is an equilibrium dispersion of oil-in-water (Type I) or water-in-oil (Type II). The hydrophilic lipophilic deviation (HLD) value measures the departure of a microemulsion's thermodynamic state from the optimum Type III (bicontinuous) formulation. Negative, zero, or positive HLD values suggest the formation of Type I, Type III or Type II microemulsions, respectively.

Determination of salt concentration which produces an equal volume of oil and volume of water within a formed microemulsion, or Type III bicontinuous formulation, may be performed using techniques not requiring complex equipment. For example, an equal volume of oil composition and aqueous composition may be combined with SAS-$C_8$ and salt. The composition may then be mixed and allowed to equilibrate. Equilibration may occur at about 25 °C. Visual inspection of the composition phase behavior may then be performed to determine if a microemulsion having an equal volume of oil and volume of water is formed. When such a microemulsion is formed, then HLD = 0. The salt concentration producing such microemulsion (may be referred to as "optimal salt concentration") may then be used to calculate the equivalent alkane carbon number (EACN). Typically, multiple compositions of varying salt concentration are used to make this determination.

Upon determination of the optimal salt concentration, Formula (II):

$$\text{Formula (II): HLD} = \ln(S) - K \cdot \text{EACN} + Cc - f(A) - \alpha_T \Delta(T)$$

may be used to determine the EACN. HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

The inventive methods are useful for application with various oil(s). In some embodiments, the oil may be a fragrance oil. In some embodiments, the oil may be another oil type. In some embodiments, the oil may be a mixture of oils. In some embodiments, the oil may contain water soluble solvents. In some embodiments, the water soluble solvent is dipropylene glycol. In some embodiments, the one or more oils are non-polar. In some embodiments, the one or more oils comprise a polar oil and a non-polar oil.

The inventive methods may utilize a surfactant or solubilizer. Suitable surfactants include neutral surfactants (such as polyoxyethylene hydrogenated castor oil or fatty acids of sugars), anionic surfactants (such as sodium lauryl sulfate), cationic surfactants (such as the ammonium cation surfactants) or zwitterionic surfactants. In some embodiments, the compositions of the present disclosure include at least one surfactant that is a polyoxyethylene (polyethylene glycol; PEG) hydrogenated castor oil. Suitable PEG-40 hydrogenated castor oils include, for example, those sold by BASF under the name Cremophor RH 410 and Cremophor RH 40. Those of skill in the art will appreciate that for PEG-40 hydrogenated castor oils having higher percentages of active, for example Cremophor RH 40, which is typically provided as a pure (i.e., 100%) composition of PEG hydrogenated castor oil, a correspondingly smaller amount of such PEG-40 hydrogenated castor oil would be employed. In some embodiments, the surfactant is anionic. In certain embodiments, the surfactant is nonionic. In certain embodiments, the surfactant is amphoteric. In certain embodiments, the amphoteric surfactant is cocamidopropyl betaine.

The mass ratio of surfactant to the compound of Formula (I) may vary. In some embodiments, the mass ratio of surfactant to the compound of Formula (I) is from about 1:1 to about 1:8. In some embodiments, the mass ratio of surfactant to the compound of Formula (I) is from about 1:2 to about 1:6. In some embodiments, the mass ratio of surfactant to the compound of Formula (I) is about 1:4.

**EXAMPLE**

A total of 5 mL fragrance oil and 5 mL of an aqueous phase containing 2 wt. % reference surfactant SAS-$C_8$(Alfoterra k2-41s, Sasol, South Africa) along with NaCl were added into a vial, hand-shaken once a day for three sequential days and allowed to equilibrate at 25 °C. Multiple vial samples were prepared, each having a particular NaCl concentration, in order to yield a range of NaCl concentrations. Visual observation was made to determine formation of a middle phase microemulsion. The NaCl concentration providing for a defined middle phase microemulsion system having an equal volume of oil in the microemulsion and water in the microemulsion is referred to as the optimal formulation and used to determine the optimal salinity (S*).

Determination of EACN was made by use of Formula (II):

$$\text{Formula (II): HLD} = \ln(S) - K \cdot \text{EACN} + Cc - f(A) - \alpha_T \Delta(T),$$

where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the surfactant used ($C_8$-$(PO)_4$-EO-$SO_4Na$), f(A) is a function of added alcohol (f(A) is 0 for formulations without alcohol), $\alpha_T$ is an empirical constant and $\Delta(T)$ is the temperature deviation from 25 °C ($\alpha_T\Delta(T)$ is 0 when at a constant temperature of 25 °C). Therefore, when experimentation is performed at constant 25 °C and without added alcohol, then f(A) - $\alpha_T\Delta(T)$ equals 0. When the optimal formulation is achieved, or an equal volume of oil in the microemulsion and water in the microemulsion exists, then HLD = 0 and S = S*. The SAS-$C_8$ surfactant was a 28.7% active solution having a k value of 0.053 and a Cc value of -2.47.

Figure 1 shows the results of testing with the Fragrance "J". NaCl concentrations from left to right are 4.6, 4.8, 5.0, 5.2, 5.4, 5.6 wt. %. A NaCl concentration of 5.0 wt. % shows the optimum concentration, therefore HLD = 0 at this NaCl concentration. Using Formula (II), EACN of Fragrance "J" was calculated to be -14.8.

Table 1 shows the average EACN values (n = 3) and standard deviation for various fragrances.

Table 1

| Fragrance | EACN | Standard Deviation |
|---|---|---|
| A | -33.3 | 2.7 |
| B | -28.3 | 0.8 |
| c | -27.6 | 0.4 |
| D | -25.5 | 1.2 |
| E | -24.3 | 1.2 |
| F | -21.5 | 0.5 |
| G | -20.0 | 1.0 |
| H | -18.1 | 0.5 |
| I | -16.4 | 0.8 |
| J | -14.8 | 0.8 |
| K | -14.8 | 0.8 |
| Limonene | -0.9 | 1.0 |
| Vitamin E | 5.2 | 0.8 |
| Isopropyl myristate | 7.8 | 0.6 |
| Flaxseed oil | 13.0 | 0.5 |
| Olive oil | 15.0 | 0.5 |

While the present invention has been described with reference to several embodiments, which embodiments have been set forth in considerable detail for the purposes of making a complete disclosure of the invention, such embodiments are merely exemplary and are not intended to be limiting or represent an exhaustive enumeration of all aspects of the invention. The scope of the invention is to be determined from the claims appended hereto. Further, it will be apparent to those of skill in the art that numerous changes may be made in such details without departing from the spirit and the principles of the invention.

The invention also refers to the following numbered embodiments, wherein the term "claim" refers to "embodiment".

1. A method for determining the equivalent alkane carbon number (EACN) of a composition comprising oil, the method comprising;

a. mixing one or more compositions comprising;

i. a compound having Formula (I) $C_a$-$(PO)_b$-$(EO)_c$-$SO4Na$, where a is from 6 to 18, b is from 1 to 12, and c is from 0 to 4;
ii. one or more oils;
iii. water; and
iv. a salt;

b. determining the salt concentration which produces an equal volume of oil and volume of water within a formed microemulsion;
c. determining the equivalent alkane carbon number using Formula (II) HLD = ln(S) - K EACN + Cc - f(A) - $\alpha_T \Delta(T)$;

where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

2. The method according to claim 1, wherein the salt is selected from sodium chloride, potassium chloride, ammonium chloride, calcium chloride, magnesium chloride, and a combination of two or more thereof.

3. The method according to claim 1 or claim 2, wherein the salt comprises sodium chloride.

4. The method according to any foregoing claim, wherein the salt is present at an amount of about 1 wt. % to about 10 wt. %.

5. The method according to any foregoing claim, wherein the salt is present at an amount of about 10 wt. % to about 25 wt. %.

6. The method according to any foregoing claim, wherein at least one of the one or more oils is non-polar.

7. The method according to any foregoing claim, wherein the one or more oils comprise a polar oil and a non-polar oil.

8. The method according to any foregoing claim, wherein at least one of the one or more compositions further comprises a solubilizer.

9. The method according to claim 8, wherein the solubilizer is pre-mixed with at least one of the one or more oils.

10. The method according to any foregoing claim, wherein at least one of the one or more oils comprises a water-soluble solvent.

11. The method according to any foregoing claim, wherein a is from 7 to 9, b is from 3 to 5, and c is from 1 to 2.

12. The method according to any foregoing claim, wherein a is 8, b is 4, and c is 1.

13. A method for determining the equivalent alkane carbon number (EACN) of a composition comprising oil, the method comprising;

a. mixing one or more compositions consisting essentially of;

i. a compound having Formula (I) $C_a$-$(PO)_b$-$(EO)_c$-SO4Na, where a is from 6 to 18, b is from 1 to 12, and c is from 0 to 4;
ii. one or more oils;
iii. water; and
iv. a salt;

b. determining the salt concentration which produces an equal volume of oil and volume of water within a formed microemulsion;
c. determining the equivalent alkane carbon number using Formula (II) HLD = ln(S) - K EACN + Cc - f(A) - $\alpha_T \Delta(T)$;

where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

14. The method according to claim 13, wherein the salt is selected from sodium chloride, potassium chloride, ammonium chloride, calcium chloride, magnesium chloride, and a combination of two or more thereof.

15. The method according to claim 13 or claim 14, wherein the salt comprises sodium chloride.

16. The method according to any one of claims 13 to 15, wherein the salt is present at an amount of about 1 wt. % to about 10 wt. %.

17. The method according to any one of claims 13 to 16, wherein the salt is present at an amount of about 10 wt. % to about 25 wt. %.

18. The method according to any one of claims 13 to 17, wherein at least one of the one or more oils is non-polar.

19. The method according to any one of claims 13 to 18, wherein the one or more oils comprise a polar oil and a non-polar oil.

20. The method according to any one of claims 13 to 19, wherein at least one of the one or more compositions further comprises a solubilizer.

21. The method according to claim 20, wherein the solubilizer is pre-mixed with at least one of the one or more oils.

22. The method of any one of claims 13 to 21, wherein the one or more oils comprises a water-soluble solvent.

23. The method according to any one of claims 13 to 22, wherein a is from 7 to 9, b is from 3 to 5, and c is from 1 to 2.

24. The method according to any one of claims 13 to 23, wherein a is 8, b is 4, and c is 1.

**Claims**

1.  A method for determining the equivalent alkane carbon number (EACN) of a composition comprising oil, the method comprising;

    a. mixing one or more compositions comprising;

    i. a compound having Formula (I) $C_a$-(PO)$_b$-(EO)$_c$-SO4Na, where a is from 6 to 18, b is from 1 to 12, and c is from 0 to 4;
    ii. one or more oils;
    iii. water; and
    iv. a salt;

    b. determining the salt concentration which produces an equal volume of oil and volume of water within a formed microemulsion;
    c. determining the equivalent alkane carbon number using Formula (II)

$$HLD = \ln(S) - K \cdot EACN + Cc - f(A) - \alpha_T \Delta(T);$$

    where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

2. The method according to claim 1, wherein the salt is selected from sodium chloride, potassium chloride, ammonium chloride, calcium chloride, magnesium chloride, and a combination of two or more thereof.

3. The method according to claim 1 or claim 2, wherein the salt is present at an amount of about 1 wt. % to about 10 wt. %.

4. The method according to any foregoing claim, wherein the salt is present at an amount of about 10 wt. % to about 25 wt. %.

5. The method according to any foregoing claim, wherein at least one of the one or more oils is non-polar.

6. The method according to any foregoing claim, wherein at least one of the one or more compositions further comprises a solubilizer.

7. The method according to claim 6, wherein the solubilizer is pre-mixed with at least one of the one or more oils.

8. The method according to any foregoing claim, wherein at least one of the one or more oils comprises a water-soluble solvent.

9. The method according to any foregoing claim, wherein a is from 7 to 9, b is from 3 to 5, and c is from 1 to 2.

10. A method for determining the equivalent alkane carbon number (EACN) of a composition comprising oil, the method comprising;

   a. mixing one or more compositions consisting essentially of;

      i. a compound having Formula (I) $C_a$-$(PO)_b$-$(EO)_c$-SO4Na, where a is from 6 to 18, b is from 1 to 12, and c is from 0 to 4;
      ii. one or more oils;
      iii. water; and
      iv. a salt;

   b. determining the salt concentration which produces an equal volume of oil and volume of water within a formed microemulsion;
   c. determining the equivalent alkane carbon number using Formula (II)

$$HLD = \ln(S) - K \cdot EACN + Cc - f(A) - \alpha_T \Delta(T);$$

   where HLD is the hydrophilic-lipophilic deviation value, S is the salt concentration producing equal volume of oil and volume of water within a formed microemulsion, K is an empirical constant, EACN is the equivalent alkane carbon number of the oil phase, Cc is a constant characterizing the hydrophilicity and lipophilicity of the compound of Formula (I), f(A) is a function of added alcohol, $\alpha_T$ is an empirical constant, and $\Delta(T)$ is the temperature deviation from 25 °C.

11. The method according to claim 10, wherein the salt is selected from sodium chloride, potassium chloride, ammonium chloride, calcium chloride, magnesium chloride, and a combination of two or more thereof.

12. The method according to claim 10 or claim 11, wherein the salt is present at an amount of about 1 wt. % to about 10 wt. %.

13. The method according to claim 10 or 11, wherein the salt is present at an amount of about 10 wt. % to about 25 wt. %.

14. The method according to any one of claims 10 to 13, wherein at least one of the one or more compositions further comprises a solubilizer; and wherein the solubilizer is pre-mixed with at least one of the one or more oils.

15. The method according to any one of claims 10 to 14, wherein a is from 7 to 9, b is from 3 to 5, and c is from 1 to 2.

4.6%    4.8%    5.0%    5.2%    5.4%    5.6%

wt. % NaCl

# FIG. 1

Europäisches
Patentamt

European
Patent Office

Office européen
des brevets

## EUROPEAN SEARCH REPORT

Application Number

EP 22 17 2383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/114559 A1 (UNIV OKLAHOMA STATE [US]; SABATINI DAVID [US] ET AL.) 7 October 2010 (2010-10-07) * paragraph [0034] – paragraph [0068] * ----- | 1-15 | INV. G01N33/28 C11B9/00 B01D17/02 |
| X | BUDHATHOKI MAHESH ET AL: "Design of an optimal middle phase microemulsion for ultra high saline brine using hydrophilic lipophilic deviation (HLD) method", COLLOIDS AND SURFACES A : PHYSIOCHEMICAL AND ENGINEERINGS ASPECTS, ELSEVIER, AMSTERDAM, NL, vol. 488, 30 September 2015 (2015-09-30), pages 36-45, XP029308888, ISSN: 0927-7757, DOI: 10.1016/J.COLSURFA.2015.09.066 * page 42, left-hand column, line 3 – page 42, left-hand column, line 14; figure 2 * ----- | 1-15 | |

TECHNICAL FIELDS
SEARCHED     (IPC)

G01N
A61Q
A23L
C11B
C11D
F17C
B01D

The present search report has been drawn up for all claims

| Place of search | Date of completion of the search | Examiner |
|---|---|---|
| The Hague | 23 September 2022 | Stavroulakis, E |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 17 2383

This annex lists the patent family members relating to the patent documents cited in  the above-mentioned European search report.
The members are as contained in the European Patent Office EDP file on
The European Patent Office is in no way liable for these particulars which are merely given for the purpose of information.

23-09-2022

| Patent document cited in search report | Publication date | Patent family member(s) | Publication date |
|---|---|---|---|
| WO 2010114559 A1 | 07-10-2010 | NONE | |

For more details about this annex : see Official Journal of the European Patent Office, No. 12/82

**EP 4 086 624 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63185655